(19)

Europäisches
Patentamt
European
Patent Office
Office européen
des brevets

(11) **EP 4 006 138 A1**

(12) **EUROPEAN PATENT APPLICATION**
published in accordance with Art. 153(4) EPC

(43) Date of publication:
**01.06.2022  Bulletin 2022/22**

(21) Application number: **20923615.7**

(22) Date of filing: **06.10.2020**

(51) International Patent Classification (IPC):
**C12M 1/32** (2006.01)   **C12M 1/12** (2006.01)
**C12M 1/26** (2006.01)   **C12M 1/34** (2006.01)

(86) International application number:
**PCT/KR2020/013582**

(87) International publication number:
**WO 2021/177530 (10.09.2021 Gazette 2021/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:   **06.03.2020   KR 20200028427**

(71) Applicant: **Quantamatrix Inc.**
**Seoul 03082 (KR)**

(72) Inventors:
• **LIM, Tae Geun**
  **Seoul 04421 (KR)**
• **KIM, Dong Young**
  **Seongnam-si Gyeonggi-do 13597 (KR)**

(74) Representative: **Prüfer & Partner mbB**
**Patentanwälte · Rechtsanwälte**
**Sohnckestraße 12**
**81479 München (DE)**

(54)   **RAPID CELL CULTURE INSPECTION DEVICE FACILITATING ACCURATE OBSERVATION**

(57)    Provided is a cell culture test device including a plurality of well units. Each of the well units includes an inlet portion through which a first fluid and a second fluid are introduced and a bottom portion including a first sub-well in which the first fluid is accommodated. A first stepped portion protrudes toward the center of the well unit along the circumference of the inner wall of the well unit such that the well unit has a lower portion whose inner width is smaller than that of an upper portion at the inlet portion side.

[FIG. 1]

## Description

### Technical Field

The present disclosure relates to a cell culture test device, and more specifically to a cell culture test device designed such that dispensed fluids are evenly distributed in wells of a chip, facilitating accurate observation.

### Background Art

Timely prescription of antibiotics for human patients infected with infectious agents greatly affects the survival of the patients. For correct antibiotic prescription, it is first necessary to determine the concentrations of specific antibiotics at which the growth of infection pathogenic strains is inhibited. This determination process is called antibiotic susceptibility testing.

According to a typical antibiotic susceptibility test, whether infectious pathogens are grown after culture in microwells containing various concentrations of a mixture of a culture medium and an antibiotic for about 18 hours is determined by transmittance measurements. A number of products are currently being developed to minimize the time required for culturing infectious pathogens and automate the testing process.

First, miniaturization of culture environments for infectious pathogens is a basic requirement for minimizing the culture time of the infectious pathogens. For such miniaturization and automation, there is a rapidly growing tendency to introduce disposable plastic plates having microscale structures into which microfluids are dispensed and which can be adjusted to desired shapes and sequences.

The present applicant has succeeded in developing an antibiotic susceptibility test system in which a microfluidic plate is introduced such that the growth of bacteria is determined by microscopic imaging. The use of this system is advantageous in that antibiotic susceptibility testing can be completed within a few hours but has problems in that fluids are not spread evenly throughout the wells of the microfluidic plate and stick to one wall of the microfluidic plate or cause bubble formation during their movement and fixation, resulting in distortion of the antibiotic susceptibility test results. Thus, there is a need for a new solution to these problems.

### Detailed Description of the Invention

### Problems to be Solved by the Invention

The present disclosure has been made in an effort to solve the above-described problems and intends to provide a cell culture test device designed such that dispensed fluids are evenly distributed in wells of a chip, facilitating accurate observation.

### Means for Solving the Problems

One aspect of the present disclosure provides a cell culture test device including a plurality of well units, each of which includes an inlet portion through which a first fluid and a second fluid are introduced and a bottom portion including a first sub-well in which the first fluid is accommodated, wherein a first stepped portion protrudes toward the center of the well unit along the circumference of the inner wall of the well unit such that the well unit has a lower portion whose inner width is smaller than that of an upper portion at the inlet portion side.

In one embodiment, the first stepped portion may have an angle of 90° or more with respect to the advancing direction of the second fluid such that the introduced second fluid is prevented from advancing vertically from the bottom portion.

In one embodiment, the first stepped portion may have a continuous edge shape along the circumference of the inner wall.

In one embodiment, the height of the first stepped portion may be smaller than the distance from the inner edge of the bottom portion to the center of the bottom portion.

In one embodiment, the height of the first stepped portion may be 0.2 mm to 4 mm.

In one embodiment, the inner edge of the bottom portion formed by the bottom portion and the inner wall of the unit may have a continuous edge shape along the circumference of the inner wall.

In one embodiment, the inner edge of the bottom portion formed by the bottom portion and the inner wall of the unit may have an angle of 70 to 120° with respect to the bottom portion.

In one embodiment, the bottom portion may include a second sub-well in which the second fluid is accommodated and an antibiotic is loaded at a specific location.

In one embodiment, the first sub-well and the second sub-well may be made smaller than the diameter of the bottom portion of the well unit such that a second stepped portion is formed.

In one embodiment, the second stepped portion may have an angle of 90° or more with respect to the advancing direction of the fluid such that the introduced fluid is prevented from advancing vertically from the bottom portion.

In one embodiment, the inner edge of the first sub-well may have a curved shape.

In one embodiment, the inner edge of the first sub-well may have a radius of curvature of 0.1 to 1 mm and a height of 0.1 to 1 mm.

In one embodiment, the inner edge of the second sub-well may have a curved shape.

In one embodiment, the first fluid may be a mixture solution of a gelling agent-containing liquid medium and a biological agent and the second fluid may be a solvent selected from water, cell culture media, dimethylformamide (DMF), and dimethyl sulfoxide (DMSO).

A further aspect of the present disclosure provides a cell analysis method using a cell culture test device having an array structure of a plurality of aligned well units, each of which includes an inlet portion through which a mixture solution of a gelling agent-containing liquid medium and a biological agent as a first fluid and a solvent selected from water, cell culture media, dimethylformamide (DMF), and dimethyl sulfoxide (DMSO) as a second fluid are introduced and a bottom portion including a first sub-well in which the first fluid is accommodated and a second sub-well in which the second fluid is accommodated and an antibiotic is loaded, wherein a first stepped portion protrudes toward the center of the well unit along the circumference of the inner wall of the well unit such that the well unit includes a lower portion whose inner width is smaller than that of an upper portion at the inlet portion side, the method including (a) introducing the first fluid into the first sub-well and gelling the mixture solution to form a solid thin film, (b) introducing the second fluid into the second sub-well to disperse or dissolve the antibiotic until the second fluid reaches the well unit over the second sub-well to come into contact with the solid thin film of the first fluid in the first sub-well such that the antibiotic is diffused into the solid thin film, and (c) observing changes of the biological agent on a single cell basis at the interface between the solid thin film of the first fluid and the second fluid.

In one embodiment, the method may further include (d) observing changes of the biological agent responding to the antibiotic on a single cell basis to determine the minimum inhibitory concentration (MIC) or minimum biofilm eradication concentration (MBEC) of the antibiotic.

**Effects of the Invention**

The rapid cell culture test device of the present disclosure is designed to facilitate accurate observation. Due to this design, exact amounts of fluids can be introduced into the rapid cell culture test device while avoiding the formation of defects such as air bubbles therein as much as possible, allowing for accurate testing compared to conventional antibiotic test devices. The rapid cell culture test device of the present disclosure uses cells immobilized in solid thin films to observe the behavior of an antibiotic, enabling rapid antibiotic susceptibility testing compared to conventional devices.

In addition, the rapid cell culture test device of the present disclosure can generally observe changes in the growth of single bacteria within several tens of minutes (usually 30 minutes) and changes in the growth of single colonies of bacteria within 4 to 6 hours. Accordingly, the rapid cell culture test device of the present disclosure can more accurately and rapidly determine the effects of a bioactive agent on a biological agent than conventional devices.

Furthermore, the rapid cell culture test device of the present disclosure can observe changes of a biological agent responding to an antibiotic on a single cell colony basis to determine the minimum inhibitory concentration (MIC) or minimum biofilm eradication concentration (MBEC) of the antibiotic, thus being very useful for biofilm assay as well as antibiotic susceptibility testing.

**Brief Description of the Drawings**

FIG. 1 illustrates one embodiment of a cell culture test device.

FIG. 2 illustrates a cross-section of well unit for a cell culture test device according to one embodiment of the present disclosure.

FIG. 3 illustrates an antibiotic susceptibility test using a first fluid and a second fluid introduced into a well unit.

FIG. 4 illustrates (a) a fluid that is not spread evenly in a portion of an inner wall due to its surface tension and (b) a cross-sectional view thereof.

FIG. 5 illustrates the contact angle for a fluid at an edge where two surfaces of a structure meet each other.

FIG. 6 illustrates the moving distance of a fluid on one surface of a structure and the moving distance of the fluid at an edge where two surfaces of the structure meet each other.

FIG. 7 schematically illustrates the moving behavior of a fluid on a stepped portion.

FIG. 8 schematically illustrates the behavior of a fluid introduced into a well unit.

FIG. 9 schematically illustrates the behavior of a fluid introduced into a well unit with or without a stepped portion.

FIG. 10 illustrates the formation of bubbles at an edge with a sharp corner after introduction of a fluid into a first or second sub-well.

FIG. 11 illustrates the movement of a fluid at an edge with a rounded corner after introduction of a fluid into a first or second sub-well.

**Mode for Carrying out the Invention**

Embodiments of the present disclosure will now be described in detail with reference to the accompanying drawings.

However, the present disclosure may be embodied in many different forms and should not be construed as limited to the embodiments set forth herein. Rather, these embodiments are provided so that this disclosure will be thorough and complete, and will fully convey the scope of the present disclosure to those skilled in the art. In the drawings, the dimensions, such as widths and thicknesses, of elements may be exaggerated for clarity. The drawings are explained from an observer's point of view. It will be understood that when an element is referred to as being "on" another element, it can be directly on the other element, or one or more intervening elements may also be present therebetween. Those skilled in the art will appreciate that many modifications can be made without departing from the spirit of the disclosure. Throughout the accompanying drawings, the same reference numerals are used to designate substantially the same elements.

On the other hand, terms used herein are to be understood as described below. While such terms as "first" and "second," etc., may be used to describe various elements, such elements must not be limited to the above terms. The above terms are used only to distinguish one element from another. For example, a first element may be referred to as a second element, and likewise a second element may be referred to as a first element.

As used herein, the singular forms "a", "an" and "the" are intended to include the plural forms as well, unless the context clearly indicates otherwise. It will be further understood that the terms "include(s)", "including", "have (has)" and/or "having", when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Respective steps of the methods described herein may be performed in a different order than that which is explicitly described. In other words, the respective steps may be performed in the same order as described, simultaneously, or in a reverse order.

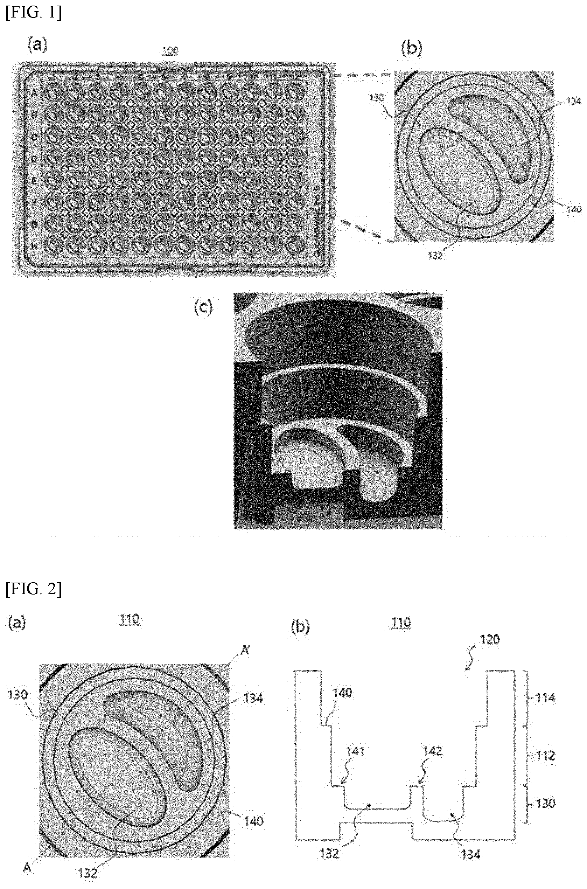

According to one aspect of the present disclosure, there is provided a cell culture test device including a plurality of well units. FIG. 1 illustrates one embodiment of the cell culture test device. In FIG. 1, (a) illustrates the constitution of the cell culture test device, (b) illustrates a partially enlarged view of the cell culture test device, and (c) illustrates a cross-sectional view of one of the well units. Referring to FIG. 1, the plurality of well units 110 are aligned in the cell culture test device. The plurality of the aligned well units 110 may have overall dimensions similar to the wells of a commercial multi-well plate. The centers of the well units 110 may coincide with the centers of the wells of a commercial multi-well plate. Referring to FIG. 2, each of the well units 110 includes an inlet portion 120 through which a first fluid and a second fluid are introduced and a bottom portion 130 including a first sub-well 132. A first stepped portion 140 protrudes toward the center of the well unit along the circumference of the inner wall of the well unit 110. The cell culture test device may optionally further include a second sub-well 134 in the bottom portion.

Multi-well plates are standard tools for processing and analyzing a large number of samples in chemical, biochemical, and/or biological assays. Multi-well plates may take various forms, sizes, and shapes. Generally, multi-well plates are designed to have standard sizes and shapes and have standard arrangements of wells. For compatibility with such multi-well plates, the plurality of well units may have standard arrangements of wells, including those found in 96-well plates (12 × 8 array of wells), 384-well plates (24 × 16 array of wells), and 1536-well plates (48 × 32 array of wells). The arrangements of the plurality of well units may be identical or similar to those of various commercially available multi-well plates. The cell culture test device 100 is readily compatible with various conventional biological analysis techniques because its dimensions are similar to those of a commercial multi-well plate. Accordingly, the well units may be arranged in a 1 × 1, 1 × 2, 1 × 4, 2 × 4, 4 × 6, 12 × 8, 24 × 16 or 48 × 32 matrix.

Each of the inlet portions 120 is typically located in the top portion of the corresponding well unit 110 and may be entirely or partially open. The inlet portion 120 may have one or more openings. The first and second fluids may be introduced into the well unit 110 through the inlet portion 120 by pipetting or injection.

The first sub-well 132 of the bottom portion 130 may be recessed in the depth direction and accommodates the first fluid introduced through the inlet portion 120. The second fluid can be directly introduced through the inlet portion 120 but at least a portion of the second fluid introduced through the inlet portion 120 is preferably accommodated in the second sub-well 134 of the bottom portion.

It is preferable to increase the cross-sectional area of the first sub-well 132 as much as possible because changes in the growth of bacteria the first sub-well 132 are observed. To this end, the first sub-well 132 may be designed to have a circular or elliptical shape in cross section and the optional second sub-well 134 may be designed to have an elliptical or crescentic shape in cross section. When each of the first sub-well 132 and the second sub-well 134 has a circular shape in cross section, the sum of the diameters of the first and second sub-wells cannot exceed the diameter of the bottom portion 130. In particular, since a predetermined amount of an antibiotic or the second fluid needs to be introduced into the second sub-well 134, the diameter of the first sub-well 132 is substantially limited to two-thirds of the diameter of the bottom portion 130. In contrast, when the first sub-well 132 has an elliptical shape in cross section, a wider field of view can be secured without the need to change the size of the second sub-well 134. When the second sub-well 134 has an oval or crescentic shape in cross section, the diameter of the first sub-well 132 can be made larger while maintaining the capacity of the second sub-well 134.

The first sub-well 132 and the second sub-well 134 may be physically separated through a partition wall 142. The partition wall 142 serves to separate the first sub-well 132 and the second sub-well 134 from each other in a horizontal direction but does not physically separate the first sub-well 132 and the second sub-well 134 from each other in a vertical direction. Accordingly, the second fluid can cross over the partition wall and be supplied to the first sub-well. Particularly, since an antibiotic is dispersed or dissolved by the second fluid and should come into contact with a solid thin film of the first fluid, it is desirable that the height of the partition wall is similar to those of the side walls of the first sub-well 132 and the second sub-well 134. A second stepped portion may be formed at the interface of the bottom portion with the first and second sub-wells. In this case, the top portion of the partition wall may also constitute the second stepped portion. The second stepped portion will be described later.

Each of the first and second fluids typically includes 70% to 99% by weight, preferably 85% to 99% by weight, most preferably 93% to 99% by weight of water as a dispersion medium or solvent. For example, the first fluid may be a mixture solution of a gelling agent-containing liquid medium and a biological agent. When the content of water in the first fluid is within the range defined above, the water can exhibit optimal performance as a dispersion medium.

The liquid medium of each of the first and second fluids may include water and may be gelled in the presence of a gelling agent. For example, the gelling agent may be agar, agarose, gelatin, alginate, collagen or fibrin. The use of agar or agarose is preferred. For example, agar may be used in an amount of 0.3 to 4% by weight in the liquid medium. The liquid medium usually requires no nutrients. In some examples, however, the liquid medium may include nutrients. The liquid medium may be a solution including a medium stimulating the division of particular cells. If the agar content is less than 0.3% by weight, the liquid medium may not be gelled. Meanwhile, if the agar content exceeds 4% by weight, the liquid medium may be excessively gelled, making it difficult to diffuse the biological agent.

Examples of suitable biological agents include viruses, bacteria, fungi, algae, protozoa, parasitic pathogens, human and mammalian cells, and biofilms. The biological agent may be a mixture of an infectious biological agent (*e.g.*, a bacterial, viral or fungal strain) and blood cells. For example, the biological agent may be blood collected from a human infected with bacteria. In this case, a medium stimulating the division of only the bacterial cells can be used to distinguish the bacterial cells from the blood cells based on their different sizes. The biological agent may grow in a liquid or solid medium and the growth of the biological agent may be affected by the kind and concentration of a foreign bioactive agent. The density of the biological agent in the mixture solution is from $10^2$ to $10^{10}$ cells/ml, preferably from $10^4$ to $10^9$ cells/ml, more preferably from $10^5$ to $10^8$ cells/ml. If the density of the biological agent is below the lower limit, it may be difficult to perceive the location of the biological agent. Meanwhile, if the density of the biological agent exceeds the upper limit, it may be difficult to perceive the individual state of the biological agent.

The bottom portion 130 or the first sub-well 132 may include an antibiotic in a solid form. In this case, the antibiotic may be in the form of a dried solid. The solvent is capable of dissolving the dried antibiotic and may be selected from water, cell culture media, dimethylformamide (DMF), and dimethyl sulfoxide (DMSO). The solvent may be introduced by pipetting such that the antibiotic in a solid form is completely dissolved and the resulting solution has a uniform concentration. Any drying process that does not deteriorate the efficacy of the antibiotic may be used without limitation to dry the antibiotic. The antibiotic is preferably dried by heat drying, freeze drying or vacuum drying, most preferably heat drying. The antibiotic thus dried is introduced into the bottom portion or the first sub-well. Alternatively, the antibiotic solution may be introduced into the bottom portion or the first sub-well and dried by the drying process mentioned above.

In the case where the cell culture test device includes the second sub-wells 134, it is preferable that a dried antibiotic is present in the second sub-wells. In this case, the second fluid is preferably introduced into the second sub-wells to dissolve the antibiotic present in the second sub-wells and fills the bottom portions 130 and lower portions 112 of the well units.

The second fluid may be a solution containing an antibiotic or a solvent dissolving an antibiotic. As a result, aliquots of the second fluid containing an antibiotic may be loaded in the second sub-wells 134. The volume of the second sub-well 134 may vary depending on a desired scale of the system but may be determined between 1 μl and 50 μl, which is an achievable range. If the volume of the second sub-well is less than 1 μl, the reduced size of the well makes pipetting and drying difficult. Meanwhile, if the volume of the second sub-well exceeds 50 μl, large amounts of

a sample and reagents are required, resulting in a reduction in efficiency.

FIG. 2 illustrates one of the well units of the cell culture test device according to the present disclosure. In FIG. 2, (a) is a top view of the well unit 110 of the cell culture test device and (b) is a cross-sectional view taken along line A-A' of (a). The first sub-well 132 may be in the form of a well that has a sufficient space to accommodate the first fluid. The volume of the first sub-well 132 is not particularly limited and may be large enough to observe responses for a long time after introduction of the first fluid. The volume of the first sub-well 132 may be, for example, 1 μl to 50 μl. If the volume of the first sub-well is less than 1 μl, the reduced size of the well is not suitable for observation. Meanwhile, if the volume of the first sub-well exceeds 50 μl, large amounts of a sample and reagents are required, resulting in a reduction in efficiency.

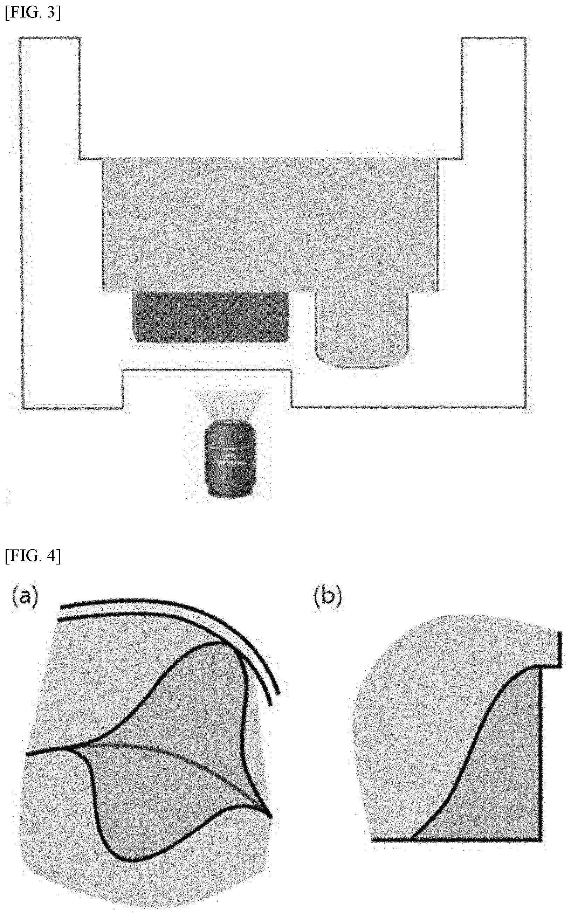

FIG. 3 illustrates an antibiotic susceptibility test using the first fluid and the second fluid introduced into the well unit.

The first fluid may be a mixture solution of a gelling agent-containing liquid medium and a biological agent. The first fluid is introduced into the first sub-well 132 and gelled. As a result of the gelling, the biological agent (*e.g.*, a bacterial strain) is immobilized in the gel matrix. Thereafter, the second fluid is introduced through the inlet portion 120 to dissolve an antibiotic. As the volume of the second fluid gradually increases, the second fluid fills the lower portion of the well unit 110 to form an interface with the gel matrix filled in the first sub-well 132. As a result, the antibiotic is diffused into the gel matrix through the interface and changes in the growth of bacteria immobilized in the gel matrix can be observed with a microscope to determine the susceptibility of the bacteria to the antibiotic.

For microscopic observation, at least a portion of the bottom face of the well unit may be made of an optically transparent material such that the target present in the first fluid is observable through the bottom face where the first sub-well 132 is located. For example, the body of the cell culture test device may be made of a transparent material. The transparent material is preferably a polymer resin such as polystyrene, polyethylene, polypropylene, polymethacrylate or polycarbonate. The cell culture test device may be manufactured by injection molding of the polymer resin.

The greatest features of the present disclosure are that the first stepped portion 140 protrudes toward the center of the well unit 110 along the circumference of the inner wall of the well unit 110 and an inner edge surrounds the bottom of the well unit. As can be seen from the cross-sectional shape of the well unit 110, the presence of the first stepped portion 140 allows the well unit 110 to have a lower portion 112 whose inner width is smaller than that of an upper portion 114 at the inlet portion side. The first stepped portion 140 may have a continuous edge shape along the circumference of the inner wall. Due to the shape of the first stepped portion 140, the behaviors of the fluids in the well unit 110 are controlled such that the fluids can be evenly spread throughout the entire well unit 110. The function of the first stepped portion 140 will be specifically described below.

Factors that have the greatest influence on the behavior of a fluid introduced into a fixed structure are the surface tension acting on the fluid and the structure surface and the gravitational force acting on the fluid as well as the inertial force. However, as the volume of a fluid decreases, the surface tension, which is affected by the surface area of the fluid rather than by the mass of the fluid, becomes a dominant force and the gravitational force has a relatively insignificant effect. Accordingly, the gravitational force has little or no effect on water and the surface tension mainly affects the behavior of the fluid in the volume range of several to several hundreds of μl, which is mainly employed in the cell culture test device of the present disclosure.

FIG. 4 illustrates (a) the fluid that is not spread evenly in a portion of the inner wall due to its surface tension and (b) a cross-sectional view thereof.

First, in the case where the second fluid is trapped in a portion of the structure and is not spread evenly, which is contradictory to its purpose, the fluid has a length similar to or greater than the width thereof and is concentrated toward the bottom rather than the top under the influence of gravity. Due to its low contact angle, the fluid has a sharp shape at the interface with the structure ((a) of FIG. 4) and is convex in cross section because it is distributed over a small area for its volume ((b) of FIG. 4).

In order for the fluid introduced into this environment to fill up the structure, as described above, it is first desirable that the contact angle of the structure surface for the fluid is maintained within 90° and it is also preferable that the surface tension of the fluid is lower than or equal to that of water, which is the strongest except those of some fluids. More specifically, it is preferable that the contact angle for the fluid is 10° to 90°. It is also preferable that the fluid has a density of 0.1 g/mL to 10 g/mL, a viscosity of 0.1 mPa/s to 10 mPa/s, and a volume of 10 μl to 200 μl. Within the density and viscosity ranges, the fluid can be completely spread in the structure. Meanwhile, if the density and viscosity of the fluid exceed the respective ranges defined above, the fluid may not be spread evenly in the structure. Here, a description will be given based on the assumption that the fluid has surface tension, density, and viscosity values similar to water (72 dyne/cm, 1 g/mL, and 1 mPa/s, respectively), a contact angle of 50°, and a volume of approximately 100 μl.

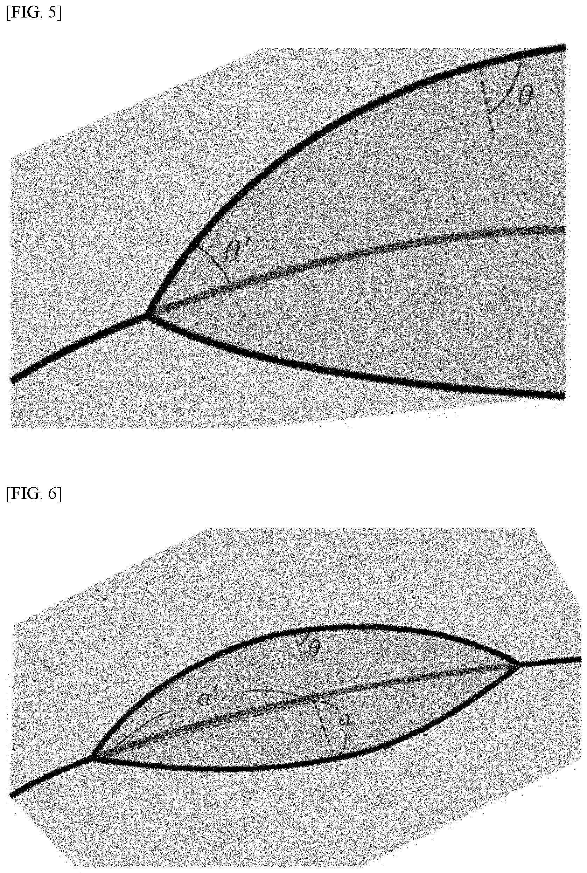

When the contact angle is about 50°, the fluid is basically spread only slightly and aggregates in a rounded shape on the surface of the well unit structure but can move farther at the inner edge of the bottom portion where two surfaces of the structure meet each other. As illustrated in FIG. 5, the contact angle between the fluid and the surface of the structure is lowered at the edge where two surfaces of the structure meet each other, allowing the fluid to spread over a longer distance.

More specifically, when the contact angle ($\theta$) between the fluid and one surface of the structure satisfies the Concus-Finn condition (for example, the contact angle ($\theta$) between the fluid and one surface of the structure is 45° or less when two surfaces of the structure form an angle of 90°), the fluid will theoretically move an infinite distance along the edge. However, if the contact angle ($\theta$) between the fluid and one surface of the structure is larger than the Concus-Finn condition and is smaller than 90°, the contact angle ($\theta$) formed by the fluid and two surfaces of the structure at the edge can be calculated by Equation 1:

[Equation 1]

$$\theta' = \cos^{-1}\left(\frac{\cos\theta}{\sin\theta}\right)\left(\frac{\pi}{4} < \theta < \frac{\pi}{2}\right)$$

where the contact angle ($\theta$) is always smaller than the contact angle ($\theta$), which can be explained by Equation 2:

[Equation 2]

$$\cos\theta' = \frac{\cos\theta}{\sin\theta} \implies \cos\theta' > \cos\theta \implies \theta' < \theta$$

The moving distance ($a$) of the fluid on one surface of the structure and the moving distance ($a'$) of the fluid at the edge where the two surfaces meet each other can be expressed by Equation 3 (see FIG. 6):

[Equation 3]

$$\frac{a'}{a} = \sqrt{\frac{1 + \frac{\cos\theta}{\sin\theta}}{1 - \frac{\cos\theta}{\sin\theta}}} \left(0 < \frac{\cos\theta}{\sin\theta} < 1\right)\left(\frac{\pi}{4} < \theta < \frac{\pi}{2}\right)$$

It is therefore preferable that the inner edge of the bottom portion formed by the bottom portion and the inner wall of the unit has an angle of 70 to 120° with respect to the bottom portion. If the angle of the inner edge is less than 70°, the size of the inlet portion is reduced, making it difficult to introduce the fluid, and the size of the well unit is reduced, making it difficult to conduct the test. Meanwhile, if the angle of the inner edge exceeds 120°, the moving distance of the fluid along the edge is shortened, making it difficult to introduce the fluid.

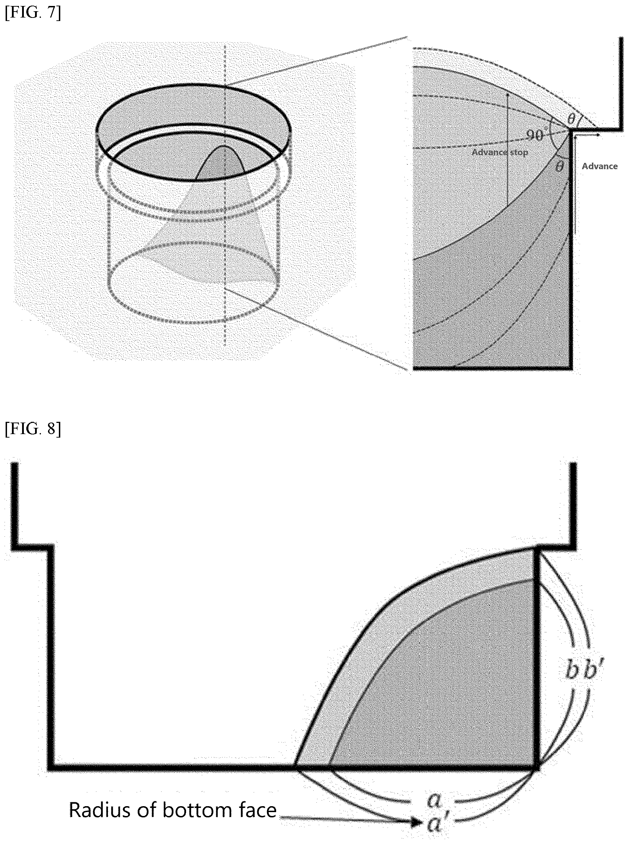

In contrast, when the fluid meets a step perpendicular to its advancing direction, its advance is stopped until its volume increases such that the contact angle increases by 90° (see FIG. 7). Based on the above condition, the present disclosure uses a step between the edged structure at the bottom face of the target space and the upper portion of the target space to achieve a uniform distribution of the fluid (FIG. 7).

More specifically, the fluid introduced into the well unit 110 climbs up the wall due to its surface tension. Here, the climbing height of the fluid is determined by a balance between the surface tension and the gravitational force of the fluid (FIG. 8). The lengths of the region covered by the fluid on the wall and the bottom face ($a$ and $b$ in FIG. 8, respectively) increase with increasing volume of the fluid and are affected by the gravitational force. Therefore, the lengths ($a$, $b$) of the region covered by the fluid on the wall and the bottom face satisfy the relationship $a \geq b$.

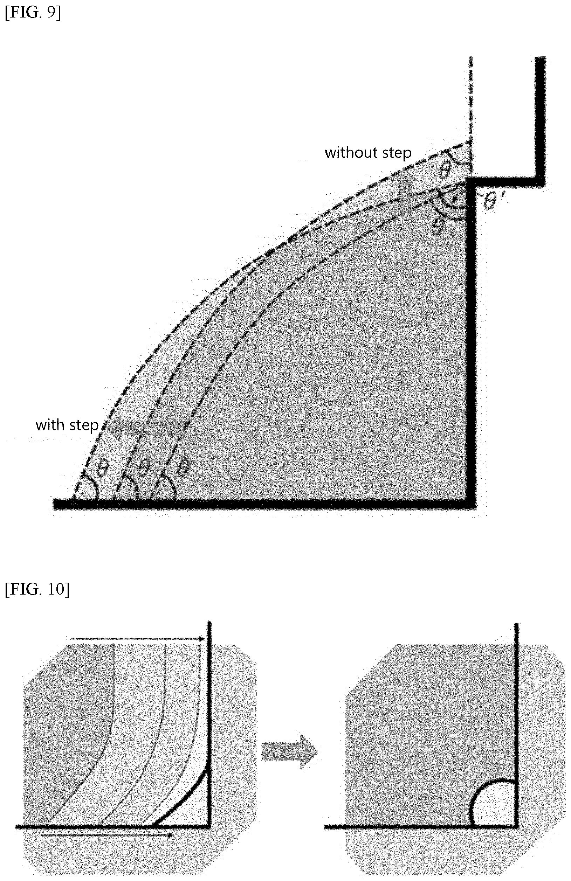

The first stepped portion plays a role in impeding the vertical advance of the fluid to reduce the surface area covered by the fluid. Another role of the first stepped portion is to increase the effect of the gravitational force, with the result that a force is applied in a direction horizontal to the fluid and a further advance of the fluid is made. As illustrated in FIG. 9, the fluid may be concentrated in the horizontal direction when a step is provided, unlike when no step is provided. Since the advance of the fluid in the presence of a step is limited to a direction opposite to the direction toward the step, the above design is preferably applied when the height ($b'$) of the first stepped portion is smaller than the radius ($a'$) of the bottom face. That is, $a'$ and $b'$ satisfy the relation of: $0 < b' < a'$.

The minimum height at which the fluid partially has a flat surface due to the gravitational force is calculated by Equation 4:

[Equation 4]

$$h = \sqrt{2\frac{\gamma}{\rho g}}$$

In the well unit whose height is equal to or greater than $h$, the fluid can be sufficiently spread even without a step. It is thus preferable that the height ($b'$) of the first stepped portion is smaller than the height ($h$) calculated by Equation 4 (that is, $b' < h$). The height ($h$) may vary depending on the kind of the fluid. Since the height ($h$) is ~4 mm for water, the height ($b'$) of the first stepped portion is preferably limited to less than 4 mm in the present disclosure using the first fluid whose height ($h$) is similar to or lower than that of water.

The height of the first stepped portion may vary depending on the volume of the second fluid. For example, assuming that the volume of the second fluid is 100 μl and the diameter of the bottom face of each well of a 96-well plate is approximately 8 mm, the height of the first stepped portion is at least 0.5 mm. Assuming that the volume of the second fluid is 10 μl and the diameter of the bottom face of each well of a 384-well plate is 4 mm, the height of the first stepped portion is 0.2 mm. It is therefore preferable that the height ($b'$) of the first stepped portion is 0.2 mm or greater.

That is, the height of the stepped portion is preferably 0.2 to 4 mm. If the height of the stepped portion is less than 0.2 mm, the second fluid reaches the top of the stepped portion, eliminating the advantage of using the stepped portion. Meanwhile, if the height of the stepped portion exceeds 4 mm, the fluid can be sufficiently spread even without the step, which also eliminates the advantage of using the stepped portion.

In the cell culture test device of the present disclosure, the presence of the stepped portion prevents the fluid from rising up above a predetermined height, and at the same time, the presence of the inner edge of the bottom portion promotes the movement of the fluid, with the result that the fluid can be dispensed evenly and quickly.

The first sub-well and the second sub-well may be made smaller than the diameter of the bottom portion of the well unit such that a second stepped portion is formed.

The movement of the fluid in the well unit may also be applied the first sub-well and the second sub-well. Particularly, since the first fluid needs to be introduced into the first sub-well, which is smaller than the well unit, the use of the stepped portions is more preferable for accurate introduction of the fluid. The first stepped portion is additionally formed in the well unit, whereas it is preferable to naturally form the second stepped portion at the interface of the bottom portion with the first and second sub-wells by making the first and second sub-wells smaller in diameter than the bottom portion of the well unit. The second stepped portion may have a continuous edge shape along the circumferences of the upper ends of the first and second sub-wells. Here, for easy observation through the first sub-well, the diameter of the first sub-well may be increased such that a portion of the side wall of the first sub-well lies in the same plane as the wall of the lower portion of the well unit. In this case, the first fluid climbs up the wall and invade the lower portion of the well unit, which may cause an error in the analysis of test results. It is therefore preferable to form the second stepped portion by making the first and second sub-wells smaller in diameter than the bottom portion of the well unit.

The sharpness of the edge is a factor that greatly increases the advancing distance of the fluid but may create an environment where air bubbles are easily trapped. Particularly, considering that bubbles formed in the bottom portion of the first fluid have a great influence on subsequent analysis, the present inventors have also paid attention to the shape of the first sub-well in which the first fluid is accommodated in order to suppress the formation of air bubbles.

The above-described physical properties of the first fluid are similar to those of the second fluid. The principles of operation of the fluids have been described under the assumption that the physical properties of the first fluid are the same as those of the second fluid. The first fluid is composed of a hydrogel that immobilizes a microbe to be tested and is assumed to have a volume of 1 μl to 50 μl in consideration of the minimum proportion of the second fluid that serves to transport nutrients and the antibiotic. If the volume of the first fluid introduced is less than 1 μl, the area of the solution is not sufficient and the size of the well is reduced, making accurate observation difficult. Meanwhile, if the volume of the first fluid introduced exceeds 50 μl, the first fluid overflows from the first sub-well or a large amount of a sample is required, resulting in low test efficiency.

First, the formation and attachment of bubbles on the bottom face is based on the following principle. In the final stage of fluid introduction, the fluid introduced in both directions along the edge meets the fluid introduced toward the wall along the bottom face. Here, when the advancing contact angle of the fluid is close to or larger than 90°, the top portion of the fluid first meets the wall to prevent air in the bottom portion of the fluid from escaping, resulting in bubble formation (see FIG. 10). Subsequently, a pressure difference is created between the bubble and the fluid that form an interface therebetween and the corresponding pressure acts on the bubble from all directions. When a portion of the bubble is in contact with the surface of the structure, the pressure acting on the bubble is directed toward the surface of the structure. This directivity prevents the bubble from floating and allows the bubble to stick to the surface of the structure. In contrast, when the advancing contact angle does not reach 90° with respect to the structure owing to the surface tension, density, and viscosity of the fluid, the bottom portion of the fluid advancing along the edge first comes into contact with the structure and then the top portion thereof comes into contact with the structure, resulting in no bubble formation.

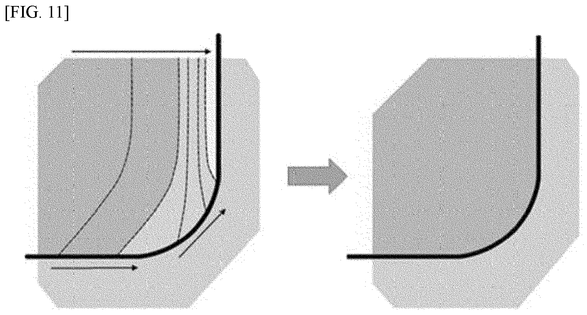

The edge may have a rounded corner. In this case, the inclination of the bottom face increases gradually and the inclination of the fluid relative to the structure decreases gradually in a state in which the advancing contact angle between the advancing fluid and the bottom face is maintained (FIG. 11). Finally, if the bottom face forms a 90° angle of inclination relative to the reference, the angle of the fluid relative to the reference is calculated by subtracting 90° from the advancing contact angle of the fluid. The calculated angle is smaller than 90° and thus meets a condition where bubbles are not formed.

Therefore, assuming that the advancing contact angle is 90° or more, the radius of the corner to avoid bubble formation should be larger than the size of bubbles formed when the edge is at a right angle. Preferably, the edge has a radius of curvature of 0.1 to 1 mm and a height of 0.1 to 1 mm. That is, the corner is rounded with a radius of curvature of 0.1 to 1 mm to a height of 0.1 to 1 mm, as illustrated in FIGS. 10 and 11. More preferably, the radius of curvature is 0.3 mm considering that it should be larger than that of bubbles when actually formed. If the curvature of the rounded edge is less than the lower limit defined above, which is smaller than the minimum curvature of bubbles, bubbles are likely to be formed. Meanwhile, if the curvature of the rounded edge exceeds the upper limit defined above, the reduced bottom area offers only a small observable field of view.

As for the shape of the second sub-well, bubbles may be formed by the introduced second fluid. In this case, the antibiotic present in the second sub-well may remain partially undissolved in the bubbles, which may affect the analysis. It is therefore preferable that the lower end edge of the second sub-well is rounded similarly to that of the first sub-well.

As described above, the use of the cell culture test device allows the dispensed fluids to be spread evenly over the entire well without bubble formation, enabling accurate imaging of changes in the growth of a target biological agent during antibiotic susceptibility testing using an optical microscope.

The rapid cell culture test device of the present disclosure may be designed to be openable and closable through a fastening structure or may be provided with a cover film made of silicone, polyethylene terephthalate (PET), polyethylene (PE), polypropylene (PP), polystyrene (PS), polycarbonate (PC), Teflon or polyvinyl chloride (PVC). A cruciform or X-shaped punch may be formed at a position of the cover film corresponding to the underlying first sub-well 120. The punch can serve as an inlet portion through which a slight amount of the first fluid is easily introduced. A cruciform or X-shaped punch may be formed at a position of the cover film corresponding to the underlying second sub-well 130. The punch can serve as an inlet portion through which the second fluid is easily introduced. In terms of convenience of use, it is preferable that the shape of the inlet portion through which the first fluid is introduced is different from that of the inlet portion through which the second fluid is introduced.

Focus marks may be provided on the underside of the cell culture test device 100 to provide information on the positions of the target. The focus marks may be marked on the underside of the cell culture test device 100 such that the target areas of the cell culture test device can be automatically tracked. The focus marks are provided in the form of microchannels by injection molding. Since the biological agent is immobilized in solid thin films, the use of the focus marks enables imaging of the same areas every time pictures are taken. The focus marks serve to correct the positions of the target in the three axial directions, *i.e.* x-, y-, and z-axis directions, in the automatic tracking of target areas.

Hereinafter, a cell analysis method of the present disclosure will be described in detail.

According to a further aspect of the present disclosure, there is provided a cell analysis method using a cell culture test device having an array structure of a plurality of aligned well units, each of which includes an inlet portion through which a mixture solution of a gelling agent-containing liquid medium and a biological agent as a first fluid and a solvent selected from water, cell culture media, dimethylformamide (DMF), and dimethyl sulfoxide (DMSO) as a second fluid are introduced and a bottom portion including a first sub-well in which the first fluid is accommodated and a second sub-well in which the second fluid is accommodated and an antibiotic is loaded, wherein a first stepped portion protrudes toward the center of the well unit along the circumference of the inner wall of the well unit such that the well unit includes a lower portion whose inner width is smaller than that of an upper portion at the inlet portion side, the method including (a) introducing the first fluid into the first sub-well and gelling the mixture solution to form a solid thin film, (b) introducing the second fluid into the second sub-well to disperse or dissolve the antibiotic until the second fluid fills the well unit on the second sub-well to come into contact with the solid thin film of the first fluid in the first sub-well such that the antibiotic is diffused into the solid thin film, and (c) observing changes of the biological agent on a single cell basis at the interface between the solid thin film of the first fluid and the second fluid.

First, a liquid medium containing a gelling agent is mixed with a biological agent to prepare a mixture solution as a first fluid.

The liquid medium may include at least about 95% by weight of water and can be gelled due to the presence of the gelling agent. The gelling agent may be, for example, agar, agarose, gelatin, alginate, collagen or fibrin. The use of agar or agarose is preferred. For example, agar may be used in an amount of 0.3 to 4% by weight in the liquid medium.

The liquid medium usually requires no nutrients. In some examples, however, the liquid medium may include nutrients. The liquid medium may be a solution including a medium stimulating the division of particular cells.

Examples of suitable biological agents include viruses, bacteria, fungi, algae, protozoa, parasitic pathogens, human and mammalian cells, and biofilms. The biological agent may grow in a liquid or solid medium and the growth thereof may be affected by the kind and concentration of a foreign bioactive agent. The density of the biological agent in the mixture solution is from $10^2$ to $10^{10}$ cells/ml, preferably from $10^4$ to $10^9$ cells/ml, more preferably from $10^5$ to $10^8$ cells/ml. If the density of the biological agent is below the lower limit, it may be difficult to perceive the location of the biological agent. Meanwhile, if the density of the biological agent exceeds the upper limit, it may be difficult to perceive the individual state of the biological agent.

Thereafter, the first fluid is supplied to each of the first sub-wells. The supplied first fluid may be evenly introduced into the first sub-well due to the curved shape of the inner edge and the second stepped portion, as described above. The first fluid can be supplied in an amount of 1 μl to 50 μl. After completion of the introduction, the first fluid is gelled to form a solid thin film immobilized with the biological agent. When the temperature of the liquid medium drops, the medium is gelled, which restricts the mobility of the biological agent. This immobilization facilitates continuous observation of the motile biological agent.

The thickness and width of the solid thin film are determined depending on the width of the first sub-well and the amount of the first fluid introduced. As used herein, the term "thin film" refers to a thin layer that has a thickness sufficient to immobilize the biological agent and to observe single cells. The thickness of the thin film is typically in the range of 1 μm to 5 mm, 1 μm to 3 mm, 1 μm to 2 mm, 1 μm to 1.5 mm, 1 μm to 1 mm, 1 μm to 800 μm, 1 μm to 500 μm, 1 μm to 100 μm, 10 μm to 3 mm, 100 μm to 500 μm, 10 μm to 1 mm, 100 μm to 1 mm, 200 μm to 1 mm or 500 μm to 1 mm, but is not particularly limited to this range. The thickness of the solid thin film may correspond to the size of a side of the solid thin film in a direction perpendicular to a side of the solid thin film to be observed. Within the thickness range of the solid thin film defined above, the biological agent immobilized in the solid thin film can be observed on a single cell basis.

Next, a solvent selected from water, cell culture media, dimethylformamide (DMF), and dimethyl sulfoxide (DMSO) is supplied as a second fluid to the second sub-well 134. The second fluid is supplied to disperse or dissolve an antibiotic loaded in the second sub-well 134. The second fluid fills up the bottom portion 130 and the lower portion 112. Thereafter, the second fluid is continuously supplied to fill the well unit. At this time, the first stepped portion ensures uniform filling of the cell unit with the second fluid while avoiding concentration of the second fluid.

After filling the second sub-well, the second fluid is supplied to the first sub-well and comes into contact with the solid thin film of the first fluid. As a result, the antibiotic dispersed or dissolved in the second fluid may diffuse into the solid thin film.

Next, responses of the biological agent to the antibiotic are observed. The biological agent immobilized in the solid thin film is distributed two-dimensionally, with the result that the biological agent can be observed on a single cell basis. According to the cell analysis method of the present disclosure, changes in the growth of the individual cells can be typically observed within several tens of minutes (normally 30 minutes). Accordingly, the cell analysis method of the present disclosure allows for the determination of the influence of the antibiotic on the biological agent in a more accurate and rapid manner than conventional methods. For example, the bioactivity of the antibiotic for bacterial cells can be tested within 3-4 hours. This rapid bioactivity test method is herein called single-cell morphological analysis (SCMA). The use of the cell culture test device enables observation of changes in single-cell morphology by time-lapse imaging under different antibiotic conditions.

An optical measurement system may be used for observation. The optical measurement system may include an imaging system, such as a CCD or CMOS camera. The optical measurement system may include optical units or devices necessary for focusing and light imaging, such as a lens, an illuminator, and a light guide. The optical measurement system may include an image processing system for processing and analyzing image data observed by the camera. The optical measurement system rapidly records and analyzes changes in the growth of the biological agent observed during testing to obtain test results. The area around the interface between the solid thin film of the first fluid and the second fluid is imaged. The imaging area may have a size of about 100 μm × 100 μm to 4000 μm × 4000 μm. It is preferable that the cross-sectional area of the first sub-well is at least larger in width than the imaging area.

Consequently, the use of the testing method based on the immobilization of the biological agent and the diffusion of the antibiotic can greatly reduce the amount of the drug and cells necessary for drug testing and enables rapid tracking of changes in the growth of single cells to obtain test results on the drug as rapidly as 2 hours (normally within 3-4 hours), compared to the prior art. This is the most rapid testing speed known thus far.

In one embodiment, the method may further include (d) observing changes of the biological agent responding to the antibiotic on a single cell basis to determine the minimum inhibitory concentration (MIC) or minimum biofilm eradication concentration (MBEC) of the antibiotic.

Biofilms are found in areas infected with microbes or to which microbes are attached. Biofilms refer to films that constitute mucilaginous microbial complexes, which are formed by microbes surrounded by polymer matrices. The formation of biofilms can greatly affect human health. Biofilms cause pulmonary infections, otitis media, periodontitis, and other infectious diseases. The resistance of bacteria present in biofilms to antibiotics is at least 1,000 times stronger than that of suspended bacteria. Flow cell systems and well-based systems have been used to investigate biofilms. However, these assay systems require a long time of several days for biofilm formation. Other difficulties associated with the use of the assay systems are that biofilms need to be stained and confocal microscopes should be used for observation. Further experiments are needed for the measurement of minimum inhibitory concentration (MIC) or minimum biofilm eradication concentration (MBEC). Such systems are very large in size and they fail to clearly show biofilm formation stages and to represent *in vivo* biofilm formation.

Thus, there is a need for efficient systems that are suitable to investigate the formation of biofilms and the reactivity of biofilms with antibiotics. In consideration of this need, the cell culture test device of the present disclosure proves to be an excellent alternative to conventional test devices.

Preferred embodiments of the present disclosure will be described in detail with reference to the accompanying drawings such that those skilled in the art can readily practice the disclosure. In describing the present disclosure, detailed explanations of a related known function or construction are omitted when it is deemed that they may unnecessarily obscure the essence of the disclosure. Certain features shown in the drawings are enlarged, reduced or simplified for ease of illustration and the drawings and the elements thereof are not necessarily in proper proportion. However, those skilled in the art will readily understand such details.

**Example 1**

A thermoplastic resin (polystyrene) was injection molded into the cell culture test device illustrated in FIGS. 1 and 2, which had an array of 12 × 8 aligned well units.

10 μl of a mixture of agarose and a bacterial solution as a first fluid was dispensed into the first sub-well of each well unit. The agarose solution was found to be evenly dispensed over the entire first sub-well by the second stepped portion. No leakage of the agarose solution from the first sub-well to the second sub-well of the well unit was observed. After completion of the dispensing into the first sub-well, the first fluid was gelled. A culture medium as a second fluid was introduced into the second sub-wells in the same manner as described for the first fluid. The same amount (100 μl) of the second fluid was added to the first stepped portion.

**Comparative Example 1**

The procedure of Example 1 was repeated except that the first stepped portions were omitted from the cell culture test device.

**Comparative Example 2**

The procedure of Example 1 was repeated except that a portion of the side wall of each first sub-well lay in the same plane as the lower portion of the corresponding well unit.

**Comparative Example 3**

The procedure of Example 1 was repeated except that the edges of the first sub-wells were at right angles.

**Test Example 1**

In each of Example 1 and Comparative Examples 1-3, 10 cell culture test devices having the same structure were manufactured (a total of 960 wells). A determination was made as to whether the first and second fluids were successfully introduced. Introduction success or failure of the fluids was judged based on bubble formation at the edges, imbalance of the second fluid, and leakage of the first fluid. The results are shown in Table 1.

[Table 1]

| | | Bubble generation at edges | Imbalance of the second fluid | Leakage of the first fluid |
|---|---|---|---|---|
| Example 1 | First fluid | 0 | - | 4 |
| | Second fluid | - | 0 | - |

(continued)

|  |  | Bubble generation at edges | Imbalance of the second fluid | Leakage of the first fluid |
| --- | --- | --- | --- | --- |
| Comparative Example 1 | First fluid | 0 | - | 6 |
|  | Second fluid | - | 52 | - |
| Comparative Example 2 | First fluid | 0 | - | 960 |
|  | Second fluid | - | 0 | - |
| Comparative Example 3 | First fluid | 462 | - | 0 |
|  | Second fluid | - | 0 | - |

As can be seen from the results in Table 1, a severe imbalance of the second fluid was observed in the test devices of Comparative Example 1, from which the first stepped portions were omitted, compared to in the test devices of Example 1. Leakage of the first fluid was observed in the test devices of Comparative Example 2 in which a portion of the side wall of each first sub-well lay in the same plane as the lower portion of the corresponding well unit to partially remove the second stepped portion. Many bubbles were observed in the test devices of Comparative Example 3 in which the edges of the first and second sub-wells were made sharp. In contrast, the formation of the first and second stepped portions and the sub-wells with rounded corners in the test devices of Example 1 enabled a more accurate introduction of the fluids. In addition, the use of the test devices of Example 1 is expected to improve the reliability of culture tests.

Although the particulars of the present disclosure have been described in detail, it will be obvious to those skilled in the art that such particulars are merely preferred embodiments and are not intended to limit the scope of the present disclosure. Therefore, the true scope of the present disclosure is defined by the appended claims and their equivalents.

**Claims**

1. A cell culture test device comprising a plurality of well units, each of which comprises an inlet portion through which a first fluid and a second fluid are introduced and a bottom portion comprising a first sub-well in which the first fluid is accommodated, wherein a first stepped portion protrudes toward the center of the well unit along the circumference of the inner wall of the well unit such that the well unit has a lower portion whose inner width is smaller than that of an upper portion at the inlet portion side.

2. The cell culture test device according to claim 1, wherein the first stepped portion has an angle of 90° or more with respect to the advancing direction of the second fluid such that the introduced second fluid is prevented from advancing vertically from the bottom portion.

3. The cell culture test device according to claim 1, wherein the first stepped portion has a continuous edge shape along the circumference of the inner wall.

4. The cell culture test device according to claim 1, wherein the height of the first stepped portion is smaller than the distance from the inner edge of the bottom portion to the center of the bottom portion.

5. The cell culture test device according to claim 4, wherein the height of the first stepped portion is 0.2 mm to 4 mm.

6. The cell culture test device according to claim 1, wherein the inner edge of the bottom portion formed by the bottom portion and the inner wall of the unit has a continuous edge shape along the circumference of the inner wall.

7. The cell culture test device according to claim 6, wherein the inner edge of the bottom portion formed by the bottom portion and the inner wall of the unit has an angle of 70 to 120° with respect to the bottom portion.

8. The cell culture test device according to claim 1, wherein the first sub-well is made smaller than the diameter of the bottom portion of the well unit such that a second stepped portion is formed.

9. The cell culture test device according to claim 1, wherein the bottom portion comprises a second sub-well in which the second fluid is accommodated and an antibiotic is loaded at a specific location.

10. The cell culture test device according to claim 1, wherein the inner edge of the first sub-well has a curved shape.

11. The cell culture test device according to claim 10, wherein the inner edge of the first sub-well has a radius of curvature of 0.1 to 1 mm and a height of 0.1 to 1 mm.

12. The cell culture test device according to claim 1, wherein the first fluid is a mixture solution of a gelling agent-containing liquid medium and a biological agent and the second fluid is a solvent selected from water, cell culture media, dimethylformamide (DMF), and dimethyl sulfoxide (DMSO).

13. A cell analysis method using a cell culture test device having an array structure of a plurality of aligned well units, each of which comprises an inlet portion through which a mixture solution of a gelling agent-containing liquid medium and a biological agent as a first fluid and a solvent selected from water, cell culture media, dimethylformamide (DMF), and dimethyl sulfoxide (DMSO) as a second fluid are introduced and a bottom portion comprising a first sub-well in which the first fluid is accommodated and a second sub-well in which the second fluid is accommodated and an antibiotic is loaded, wherein a first stepped portion protrudes toward the center of the well unit along the circumference of the inner wall of the well unit such that the well unit comprises a lower portion whose inner width is smaller than that of an upper portion at the inlet portion side, the method comprising (a) introducing the first fluid into the first sub-well and gelling the mixture solution to form a solid thin film, (b) introducing the second fluid into the second sub-well to disperse or dissolve the antibiotic until the second fluid fills the well unit on the second sub-well to come into contact with the solid thin film of the first fluid in the first sub-well such that the antibiotic is diffused into the solid thin film, and (c) observing changes of the biological agent on a single cell basis at the interface between the solid thin film of the first fluid and the second fluid.

14. The cell analysis method according to claim 13, further comprising (d) observing changes of the biological agent responding to the antibiotic on a single cell basis to determine the minimum inhibitory concentration (MIC) or minimum biofilm eradication concentration (MBEC) of the antibiotic.

[FIG. 1]

(a) 100

(b)

130
134
140
132

(c)

[FIG. 2]

(a) 110

A'
130
134
140
A
132

(b) 110

120
140
141
142
114
112
130
132
134

[FIG. 3]

[FIG. 4]

(a)

(b)

[FIG. 5]

[FIG. 6]

[FIG. 7]

[FIG. 8]

Radius of bottom face

[FIG. 9]

[FIG. 10]

[FIG. 11]

<div style="text-align:center">

**INTERNATIONAL SEARCH REPORT**

</div>

| International application No. |
|---|
| **PCT/KR2020/013582** |

**A.    CLASSIFICATION OF SUBJECT MATTER**

   **C12M 1/32**(2006.01)i; **C12M 1/12**(2006.01)i; **C12M 1/26**(2006.01)i; **C12M 1/34**(2006.01)i

According to International Patent Classification (IPC) or to both national classification and IPC

**B.    FIELDS SEARCHED**

Minimum documentation searched (classification system followed by classification symbols)

   C12M 1/32; C12M 1/00; C12M 1/12; C12M 1/18; C12M 3/00; G01N 33/50; C12M 1/26; C12M 1/34

Documentation searched other than minimum documentation to the extent that such documents are included in the fields searched

   Korean utility models and applications for utility models: IPC as above
   Japanese utility models and applications for utility models: IPC as above

Electronic data base consulted during the international search (name of data base and, where practicable, search terms used)

   eKOMPASS (KIPO internal) & keywords: 세포배양(cell culture), 웰(well), 서브웰(sub well), 단차(stepped)

**C.    DOCUMENTS CONSIDERED TO BE RELEVANT**

| Category* | Citation of document, with indication, where appropriate, of the relevant passages | Relevant to claim No. |
|---|---|---|
| X<br><br>Y | JP 2016-182091 A (NATIONAL INSTITUTE OF ADVANCED INDUSTRIAL SCIENCE AND TECHNOLOGY et al.) 20 October 2016. See paragraphs [0009], [0012] and [0015]; and figure 3. | 1-8,10,11<br><br>9,12-14 |
| Y | KR 10-1711105 B1 (QUANTAMATRIX INC.) 06 March 2017. See paragraph [0048]; and claims 1, 9-13, 15 and 18. | 9,12-14 |
| A | KR 10-2013-0058954 A (SAMSUNG ELECTRO-MECHANICS CO., LTD.) 05 June 2013. See entire document. | 1-14 |
| A | JP 2018-143168 A (COORSTEK KK) 20 September 2018. See entire document. | 1-14 |

☑ Further documents are listed in the continuation of Box C.          ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **13 January 2021** | **15 January 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2020/013582**

**C.    DOCUMENTS CONSIDERED TO BE RELEVANT**

| Category* | Citation of document, with indication, where appropriate, of the relevant passages | Relevant to claim No. |
|-----------|-----------------------------------------------------------------------------------|----------------------|
| PX | KR 10-2145842 B1 (QUANTAMATRIX INC.) 19 August 2020. See entire document. | 1-14 |

Form PCT/ISA/210 (second sheet) (July 2019)

INTERNATIONAL SEARCH REPORT
Information on patent family members

International application No.

**PCT/KR2020/013582**

| Patent document cited in search report | | | Publication date (day/month/year) | Patent family member(s) | | | Publication date (day/month/year) |
|---|---|---|---|---|---|---|---|
| JP | 2016-182091 | A | 20 October 2016 | JP | 6521432 | B2 | 29 May 2019 |
| KR | 10-1711105 | B1 | 06 March 2017 | EP | 3434370 | A4 | 30 October 2019 |
| | | | | EP | 3434370 | A1 | 30 January 2019 |
| | | | | US | 2019-0100786 | A1 | 04 April 2019 |
| | | | | WO | 2017-183875 | A1 | 26 October 2017 |
| KR | 10-2013-0058954 | A | 05 June 2013 | US | 2013-0133778 | A1 | 30 May 2013 |
| JP | 2018-143168 | A | 20 September 2018 | None | | | |
| KR | 10-2145842 | B1 | 19 August 2020 | None | | | |

Form PCT/ISA/210 (patent family annex) (July 2019)